# EUROPEAN PATENT SPECIFICATION

(11) **EP 2 644 193 B1**
(45) Date of publication and mention of the grant of the patent: **08.10.2014**
(21) Application number: 12174524.4
(22) Date of filing: 29.06.2012
(51) Int. Cl.: A61K 9/70, A61K 8/27, A61K 8/34, A61K 8/41, A61K 8/44, A61K 8/891, A61K 8/92

(54) **Topical formulations for the prevention of sexually transmitted disease and methods of producing the same**
Topische Formulierungen zur Vorbeugung von sexuell übertragbaren Krankheiten und Verfahren zu deren Herstellung
Formulations topiques pour la prévention de maladies sexuellement transmissibles et leurs procédés de production

(30) Priority: 26.03.2012 US 201213430267
(43) Date of publication of application: 02.10.2013
(73) Proprietor: CLJI I.P. Company LLC, North Palm Beach, FL 33408 (US)
(72) Inventor: Lichtblau, Craig, Jupiter, Florida 33458 (US); Iparraguirre, Jose I, Miami, Florida 33176 (US)
(74) Representative: HGF Limited

(56) References cited:
- US-B2- 8 062 631

## Description

### FIELD OF THE INVENTION

This invention relates to topical protective formulations for use in the prevention of the spread of sexually transmitted diseases (STDs); and particularly to unique topical formulations including a plurality of film forming excipients, which act in concert to provide a barrier to help inhibit the transmission of STDs.

### BACKGROUND OF THE INVENTION

Sexually transmitted diseases (STDs) are a universal concern, effecting millions of individuals and straining health care systems. More than 20 different sexually transmitted diseases have been identified by the medical community and generally fall into two groups, including bacterial types (e.g., gonorrhea, chlamydia and syphilis) and viral types (human immunodeficiency virus (HIV), human papilloma virus (HPV) and hepatitis). The numerous diseases affect men, women and children of all backgrounds, races and economic classifications. Despite years of research and educational programs, the transmission of sexually transmitted diseases remains a global health threat. Although specific methods of transmission may vary depending on the disease-causing organism, STDs are usually transmitted to the uninfected person through injured or exposed skin or mucous membranes during sexual contact.

Treatments may be available for some types of STDs (e.g. antibiotic treatment for gonorrhea or chlamydia). However, most people who suffer from these types of STDs are unaware that they have the disease and therefore do not get the necessary treatment. Moreover, because of the sociological impact and generally negative stigma associated with these diseases, people are reluctant to seek such treatments. The continued emphasis on educating the population as to the use of "mechanical barriers" such as condoms has helped to decrease the morbidity of most STDs but more preventative methods need to be developed to prevent STD transmission.

Chemical actives such as microbicides, antimicrobials, and spermicides, most notably, nonylphenoxypoly(ethyleneoxy)-ethanol (also referred to as nonoxynol-9) have been used in topical formulations to effectively reduce the rate of STD transmission, especially when used in conjunction with prophylactics. However, many of these chemical actives are harsh and have been shown to induce local irritation, inflammation, and ulcerations, which might actually favor the transmission of STDs. Thus, a need exists for topical formulations that do not cause irritation and will help inhibit the spread of STDs, especially when used in combination with condoms, and thereby provide an additional degree of protection from contamination, should the condom become damaged.

### PRIOR ART

Although there are numerous patents and publications directed to formulations containing chemical actives, such as, microbicides, antimicrobials, spermicides, and drug delivery carriers (liposomes, micelles), none of the known prior art teach formulations comprising non-irritating agents that provide a physical barrier to the permeation of pathogens.

U.S. Patent Application 2003/0143189 A1 to Askill et al., is directed to a method of treating skin lesions by forming a polymeric film over the lesions to inhibit proliferation of infectious agents in the lesions. These compositions also include one or more chemical agents in combination therewith.

U.S. Patent 6,835,717 to Hildreth discloses a method of reducing the risk of a sexually transmitted pathogen by contacting a pathogen within the composition which consists of β-cyclodextrin.

U.S. Patent No. 6,821,958 to Hershline discloses a method of preventing viral transmission using an alkylsulfate derivative of sulfated dextrin as a topical formulation.

U.S. Patent 6,582,711 to Asmus et al., discloses an antimicrobial hydroalcoholic composition having a cationic polymeric thickener.

U.S. Patent No. 6,355,235 to Cone et al., consists of an antibody capable of trapping sperm and a pharmaceutical carrier.

U.S. Patent No. 6,328,991 to Myhling discloses a chemical composition to prevent the transmission of sexually transmitted diseases comprising nonylphenoxpoly-(ethyleneoxy)-ethanol, benzalkonium chloride and povidone iodine.

U.S. Patent 5,439,685 to Augros is a composition of an agent active against microorganisms responsible for sexually transmitted diseases together with a film-forming agent such as dimethylpolysiloxane, and benzalkonium chloride as a spermicidal.

U.S. Patent 4,952,411 to Fox, Jr. et al., is a composition of silver sulfadiazine, alone or in combination with chlorhexidine or sodium deoxycholate (antimicrobial and detergent).

### SUMMARY OF THE INVENTION

The purpose of this invention is to provide non-irritating protective formulations to be used as an adjunct in preventing the spread of a broad range of sexually transmitted diseases. The products of the present invention can be formulated as a topical lotion, cream, solution, emulsion, or the like, and will be hereinafter referred to as creams. Applicant's previous patent demonstrated that the use of antimicrobial/antiviral active agents in conjunction with film-forming excipients in the following formulations possessed unique skin protective barrier properties with enhanced persistence that inhibit "skin to skin" and "sore to sore" transmission of pathogens (e.g., viruses, bacteria, fungi, parasites, ectoparasites and mycoplasmas) linked to communicable diseases.

Applicants have now discovered that equivalent skin protective barrier properties, with enhanced persistence, which are also effective in providing the inhibition of "skin to skin" and "sore to sore" transmission of pathogens (e.g., viruses, bacteria, fungi, parasites, ectoparasites and mycoplasmas) linked to communicable diseases, can be realized in the absence of an antimicrobial/antiviral agent.

Skin protectant products are regulated under CFR 21

Part 347 "Skin Protectant Drug Products for Over the Counter Human Use". The official description of a skin protectant is "a drug product that temporarily protects injured or exposed skin or mucous membrane surfaces from harmful or annoying stimuli, and may help provide relief to such surfaces." These regulations cover applicable ingredients, as well as labeling requirements for over the counter skin protectants. Active ingredients officially classified as skin protectants compositions as well as certain combinations of these compositions are listed in the CFR 21 Sec. 347.10, reproduced in Table 1 below. In accordance with the instant invention, the phrase "a skin protectant composition provided in a skin protecting effective concentration" will be understood to mean any of the following ingredients within their designated range of concentrations:

These include any of the following within the concentration range specified:

**TABLE 1**

| **Ingredient** | **Concentration** |
|---|---|
| Allantoin | 0.5 to 2.0 % |
| Aluminum hydroxide gel | 0.15 to 5.0% |
| Calamine | 1.0 to 25.0 % |
| Cocoa Butter | 50.0 to 100.0% |
| Cod liver oil | 5.0 to 13.56 % (in accordance with Sec. 347.20(a)(1) or (a) (2), provided the product is labeled so that the quantity used in a 24-hr period does not exceed 10,000 USP Units vitamin A and 400 USP Units cholecalciferol. |
| Colloidal oatmeal | 0.007 minimum; 0.003 minimum in combination with mineral oil in accordance with Sec. 347.20(a)(4). |
| Dimethicone | 1.0 to 30.0% |
| Glycerin | 20.0 to 45.0% |
| Hard fat | 50.0 to 100.0% |
| Kaolin | 4.0 to 20.0% |
| Lanolin | 12.5 to 50.0% |
| Mineral oil | 50.0 to 100.0%; 30.0 to 35.0% in combination with colloidal oatmeal in accordance with Sec. 347.20(a)(4). |
| Petrolatum | 30.0 to 100.0% |
| Topical starch | 10.0 to 98.0% |
| White petrolatum | 30.0 to 100.0% |
| Zinc acetate | 0.1 to 2.0% |
| Zinc carbonate | 0.2 to 2.0% |
| Zinc oxide | 1.0 to 25.0% |

It has been discovered that incorporation of at least one of the aforementioned skin protectants compositions listed in Table 1 in combination with other film forming excipients, in particular, film-forming emollients (e.g., Cetostearyl alcohol, Cetyl alcohol), silicone-containing excipients/skin protectants (e.g., polydimethylsiloxane derivatives of varying viscosities, utilized either singly or in combination, and marketed under names such as Dimethicone 20 and Dimethicone 12500), emulsifying agents (e.g. selected from Cetearyl alcohol (a mixture of fatty alcohols, predominantly stearyl and cetyl alcohols, polyoxyethylene ethers of a mixture of high molecular mass saturated fatty acids (mainly cetyl alcohol and stearyl alcohol, having a number of ethylene oxide residues in the polyoxyethylene chain, e.g. 20, 12 or the like, and referred to as Ceteareth-20, Ceteareth-12, or the like, and/or mixtures thereof), humectants (e.g. glycerin and anhydrous lanolin) and chelating compounds(e.g. disodium edetate) in a formulation for topical application results in a product that temporarily protects injured or exposed skin or mucous membrane surfaces from harmful or annoying stimuli, thereby preventing cross-contamination of pathogenic microorganisms responsible for sexually transmitted diseases (e.g., Herpes simplex virus type 1 and type 2 (HSV-1, HSV-2), human immunodeficiency virus (HIV), or the like) through skin or mucous membrane surfaces. The novel formulation products of this invention are persistent in that they form a film or barrier on healthy or even damaged skin.

The hydrophobic portions of the instant formulations utilize a combination of film-forming excipients, including skin protectant(s) listed in Table 1, silicones and silicone derivatives or like equivalents, and film-forming emollients. The emulsifiers throughout the continuous aqueous phase disperse these ingredients. They liquefy and spread over the skin as a result of exposure to body heat. This forms a physical hydrophobic layer, which resides on the skin surface (including mucosal membranes) and provides a barrier, which would inhibit penetration of liquids, and pathogens that are primarily hydrophilic in nature. When used in combination with consistent and careful use of condoms, the products of the present invention help inhibit the spread of STDs and protect the skin from contamination should the condom become damaged.

Accordingly, it is an objective of the instant invention to teach various topical protective formulations to be used in preventing the spread of a broad range of sexually transmitted diseases.

Another objective of the present invention is to teach formulations which are condom compatible, meaning that they are latex friendly and provide effective lubricity.

It is yet another objective of the instant invention to teach skin protective formulations wherein contact with the skin results in the formation of a hydrophobic skin protective surface layer.

It is yet another objective of the instant invention to teach skin protective formulations wherein contact with the skin results in the formation of a mechanical barrier skin protective surface layer.

Yet another objective of the invention is to teach products formulated as a lotion, cream, solution, emulsion, or other topically applied product.

Other objects and advantages of this invention will become apparent from the following description, wherein are set forth, by way of illustration and example, certain embodiments of this invention.

### DETAILED DESCRIPTION OF THE INVENTION

Detailed embodiments of the instant invention are disclosed herein, however, it is to be understood that the disclosed embodiments are merely exemplary of the invention, which may be embodied in various forms. Therefore, specific functional and structural details disclosed herein are not to be interpreted as limiting, but merely as a basis for the claims and as a representation basis for teaching one skilled in the art to variously employ the present invention in virtually any appropriately detailed structure.

Zinc oxide is an inert, non-toxic chemical compound with the chemical formula of ZnO. Zinc Oxide can be used as a skin barrier. When applied to skin, Zinc Oxide acts as a mechanical barrier that physically excludes isolates and prevents the skin from any contact with harmful stimuli. Zinc Oxide is often used in creams or lotions and provides a continuous barrier, which also helps prevent the loss of active ingredients due to friction and rubbing. Because of its inert, non-toxic characteristics and general non-solubility in water, it can be applied to skin as many times as may be needed. While zinc oxide is the preferred skin protectant composition of the present invention, other suitable skin protectant compositions and their amounts effective to provide skin protection as listed in Table 1 above could be used herein (e.g., Dimethicone).

Glycerin (INCI name: Glycerin) is a non-irritating humectant and film former. It is also water-soluble. Moreover, glycerin is compatible with latex products and provides extended lubricity, which makes it a common base for many products designed for genital use.

Lanolin is a humectant isolated from wool-bearing animals such as sheep. It is a product of the sebaceous gland and consists mostly of a mixture of cholesterol and the esters of several fatty acids. It has many commercial uses, including within the medical and cosmetic industries.

Benzalkonium Chloride is a member of the quaternary ammonium compounds. These compounds are a group of ammonium salts in which organic radicals have been substituted for all four hydrogens of the original ammonium cation. Benzalkonium Chloride has been reported to be an effective anti-viral wetting agent in the prevention of transmission of viral diseases such as HIV and herpes simplex virus. Benzalkonium Chloride is also listed in the FDA Topical Antimicrobial Drug Products Monograph as a First Aid Antiseptic.

The phrase "film-forming emollient" refers to any excipient suitable for cosmetic and pharmaceutical applications, which forms a water-repelling film. According to a preferred, albeit non-limiting embodiment, the film-forming emollients are cetyl alcohol and cetostearyl alcohol. Cetyl alcohol (IUPAC name of 1-hexadecanol) is a member of the alcohol class of compounds. It is a solid organic compound and belongs to a group of fatty alcohols. In addition to its use as an emollient, it is often used in the cosmetic industry as a surfactant in shampoos, emulsifiers or thickening agents in the manufacture of skin creams and lotions. Cetostearyl alcohol is a blend of cetyl alcohol and stearyl alcohol, two fatty alcohols derived from vegetable sources.

The phrase "silicone-containing excipient" refers to any silicone or silicone derivatives, including silicone-based skin protectants, suitable for cosmetic and pharmaceutical applications, which acts as an emollient and forms a water-repelling film, including silicone oils. In addition to skin barriers, silicone and silicone oils are highly substantive on the skin. As a result of this property, silicon and silicone oils are often used in topical formulations, such as creams and lotions, to improve the substantivity of active ingredients on the skin.

After applying the topical formulations to the skin and removing volatile substances, the film formed as a result of using a silicone-containing excipient helps to maintain the active ingredient in close contact with the skin and prevents the loss of the active ingredient by abrasion. The ability to form hydrophobic films, which can easily be applied, and spread over skin accounts for high resistance to wash-off and rub-off. Use of silicones in topical formulations over petroleum-based products is more desirable because silicones do not exhibit the negative aesthetics of petroleum and, unlike petroleum, are known to be compatible with the materials used to manufacture condoms. According to one non-limiting embodiment, Dimethicone (preferably Dimethicone 20 cSt and Dimethicone 12500 cSt) is preferred. Dimethicone is a highly pure, non-volatile silicone fluid, which is colorless and odorless. It can be used as a lubricant and emollient skin protectant.

Ceteareth-12 is part of a family with the INCI name of Ceteareth-n and refers to polyoxyethylene ethers of a mixture of high molecular mass saturated fatty alcohols. The "n" indicates the average number of ethylene oxide residues in the polyoxyethylene chain. Ceteareth-12 is a non-toxic surfactant, which is frequently used as an emulsifier in the cosmetic industry. The mixture of Cetearyl alcohol and Ceteareth-20 (EMULGADE 1000NI, Cognis Corporation) is a non-ionic, self-emulsifying base commonly used in the production of oil/water creams and lotions.

Disodium Edetate, having the chemical name of disodium (ethylene-dinitrilo) tetra-acetate dihydrate, is also commonly known as the disodium salt of EDTA. The chemical acts as a chelating compound by preventing ingredients from binding to trace elements that may be present.

Excipients useful in forming the skin protective creams, according to the present invention are described in the following non-limiting example.

The invention includes use of a formulation as herein described for the inhibition of transmission of a sexually transmitted disease.

The invention includes a formulation as herein described for use in the inhibition of transmission of a sexually transmitted disease.

As used herein a sexually transmitted disease may be for example a bacterial sexually transmitted disease, such as gonorrhea, chlamydia or syphilis, or a viral sexually transmitted disease, such as human immunodeficiency virus (HIV), human papilloma virus (HPV) or hepatitis.

### EXAMPLE 1

In formulating a batch of a skin protective cream according to the invention, active ingredients and excipients useful in the manufacture of this product, a particularly effective product resulted when ingredients were utilized within the following approximate ranges:

| **ACTIVE INGREDIENTS/EXCIPIENT** | | **WT/WT % RANGES** |
|---|---|---|
| Water phase | | |
| (1) | DEIONIZED WATER | Q.S. |
| (2) | EDETATE DISODIUM | 0.01-0.1 |
| (3) | ZINC OXIDE | 1.0-8.0 |
| (4) | GLYCERIN (USP) | 2.0-10.0 |

| Oil Phase | | |
|---|---|---|
| (5) | DIMETHICONE 20 | 10.0-20.0 |
| (6) | DIMETHICONE 12500 | 3.0-10.0 |
| (7) | CETEARYL ALCOHOL and/or CETEARETH- | 20 6.0-10.0 |
| (8) | CETEARETH-12 | 0.5-3.0 |
| (9) | ANHYDROUS LANOLIN | 0.5-3.0 |
| (10) | CETOSTEARYL ALCHOHOL | 1.0-5.0 |
| (11) | CETYL ALCOHOL | 1.0-5.0 |

A preferred, albeit non-limiting procedure for manufacture of the formulation comprises the steps of:

### Procedure:

1. Heat to 75°C -Dimethicone 20⁻, Dimethicone 12500, Cetearyl Alcohol and Ceteareth-20 (available as EMULGADE 1000 NI from Cognis), Ceteareth-12 (available as (EUMULGIN B1 from Cognis), Anhydrous Lanolin, Cetostearyl alcohol and Cetyl alcohol. Allow the materials to melt.
2. Dissolve Disodium Edetate in the deionized water (USP).
3. Heat Step #2 to 75-78 °C.
4. Disperse the Zinc Oxide in the Glycerin to form a homogeneous dispersion.
5. With high speed mixing, slowly add the materials from Step #3 (Water phase) to the materials from Step #1 (Oil phase). Initiate cooling.
6. When at 60 °C; add the dispersion from Step#4 to the materials from Step #5 (Emulsion).
7. When at 50 °C, reduce mixing speed.
9. When at 40 °C, further reduce the mixing speed.
10. When at 25°C stop mixing.

While not wishing to be bound to any particular theory, it is believed that these film forming excipients and active ingredient form a neutral and hydrophobic layer barrier which is also believed to interact with the skin thereby having a stabilizing effect upon the hydrophobic layer, which results in the enhanced persistence of the product. The use of a silicone-containing skin protectant, e.g. Dimethicone 20 and Dimethicone 12500, or a like equivalent, acts in coordination with at least one of the skin protectants compositions as defined in CFR 21 Sec. 347.10, e.g. Zinc Oxide, or others from Table 1, excipients and the film-forming emollients, and melts when contacted with the heat of the body. This in turn forms a physical hydrophobic layer that provides a barrier, which appears to inhibit penetration of liquids, and sexually transmitted pathogens (viral, bacterial) which are primarily hydrophilic in nature. This property helps protect the user from contamination due to sexual contact with infected individuals.

In order to ascertain any differences between the use of the formulation as described herein, and a formulation additionally containing an antiviral, e.g. benzalkonium chloride, the following experiment was carried out.

**TABLE 1**

| **SKIN BARRIER FORMULATION** | |
|---|---|
| | % (w/w) |
| Dimethicone 20 (Dow coming Q7-9120 Silicone Fluid | 15.030060120 |
| Dimethicone 12500 (Dow Corning Q7-9120 Silicone Fluid 12500 I cSt). | 5.010020040 |
| Zinc Oxide, USP | 2.004008016 |
| Cetearyl Alcohol & Ceteareth-20 (Emulgade | 8.016032064 |
| Glycerin, USP | 5.010020040 |
| Ceteareth-12 (Eumulgin B1, Cognis) | 1.002004008 |
| Edetate Disodium, USP | 0.050100200 |
| Anhydrous Lanolin, USP | 1.002004008 |
| Cetostearyl Alcohol, NF | 3.006012024 |
| Cetyl Alcohol, NF | 2.004008016 |
| Purified Water USP | 57.865731460 |
| | 100.00 |

In accordance with the teachings of the present invention, the skin barrier formulation illustrated in Table 1 was prepared. Additionally, a second formulation was prepared, to which the antiviral ingredient Benzalkonium Chloride (Benzalkonium Chloride 50% Solution,NF) was added in an amount of 0.20% w/w.

A test protocol was followed in order to evaluate the barrier function of the cream product against fluid and virus transmission alone and with the addition of Benzalkonium Chloride. The challenge virus selected was Herpes Simplex Virus Type 2, ATCC VR-734.

Three pieces of filter paper were weighed and placed onto a sterile glass Petri dish. Following, a piece of porous membrane (pore size 10 pm) was placed on top of the filter paper layer [except for Filter Paper Only (No Membrane) Control]. The carrier was pre-incubated at 37±1°C (Actual, 37°C) for 10±1 minutes (Actual, 10 minutes) with the lid off prior to further manipulation.

To determine efficacy as a viral barrier, 1.00 g of test agent was spread evenly across the upper side of the membrane area. Thereafter, 0.2 mL of the challenge virus was applied evenly to the treated membrane. The carrier was covered, sealed in parafilm, and incubated at 37±1°C (Actual, 37°C) for 30±1 minutes (Actual, 30 minutes).

To determine efficacy as a moisture barrier, during the carrier preparation/pre-incubation step, 1.00 g of test agent was spread evenly across the upper side of the membrane area. Following, the carrier was incubated at 37±1°C (Actual, 37°C) for 10±1 minutes (Actual, 10 minutes). Thereafter, 0.2 mL of cell culture medium was applied evenly to the treated membrane. The carrier was covered, sealed in parafilm, and incubated at 37±1°C (Actual, 37°C) for 30±1 minutes (Actual, 30 minutes). It was assayed for moisture adsorption only.

To determine moisture penetration of the filter paper, after the contact time, the membrane - if applicable, was carefully lifted and removed. The filter paper layer was weighed to measure moisture adsorption.

To determine viral penetration, the filter paper layer was placed in a 50-mL sterile tube with 15-mL of recovery medium. The filter paper layer was squeezed with a cell scraper. Following, the tube was vortexed on a vortex-type mixer for 1-2 minutes and held at room temperature for about 5 minutes. The tube was then vortexed using a vortex-type mixer for an additional 1-2 minutes; it was then centrifuged at 5000 rpm for 5 minutes. The supernatant was collected and passed through a 0.45 pm syringe filter prior to use in diluting and plating.

### Media and reagents:

RPMI 1640 + 5% Newborn Calf Serum + 0.5% Polysorbate 80
RPMI 1640 + 5% Newborn Calf Serum
Filter paper, ashless circles, 47 mm diameter, Whatman™ No. 41
10-µm membrane filters, circles, 47 mm diameter, type TCTP

The results are summarized in Table 2, wherein it is seen that the efficacy of the comparative formulations with and without Benzalkonium Chloride were equivalent in their ability to block the penetration of both viral and moisture challenges.

**TABLE 2**

| Sample ID | Cream Dosage | Input Virus* (Logᵢₒ TCID₅₀) | Virus Recovered* (Log_{i°} TC10₅₀) | Virus Reduction* (Log-ₜₒ TCI0₅₀) | Volume of virus/ media* (mL) | Normalized Moisture gain^{a} (g) |
|---|---|---|---|---|---|---|
| Skin Barrier Formula of Table Number 1-Virus Test Sample | - 1.00g | 4.33 ± 0.36 | ≤3.01 ** | ≥1.32 | 0.2 mL (virus) | 0.00 |
| Skin Barrier Formula 1 plus Benzalkonium Chloride - Virus Test Sample | - 1.00g | 4.71 ± 0.38 | ≤3.01 ** | ≥1.70 | 0.2 mL (virus) | 0.02 |

| | | | | | | |
|---|---|---|---|---|---|---|
| * Results represent the averaged data from three replicates. **A complete blockade of virus was observed in all three replicates. a Normalized based on the moisture gain from a Cream-only (no virus or media) sample | | | | | | |

It is to be understood that while a certain form of the invention is illustrated, it is not to be limited to the specific form or arrangement herein described and shown. It will be apparent to those skilled in the art that various changes may be made without departing from the scope of the invention and the invention is not to be considered limited to what is shown and described in the specification and drawings/figures.

All patents and publications mentioned in this specification are indicative of the levels of those skilled in the art to which the invention pertains. It is to be understood that while a certain form of the invention is illustrated, it is not to be limited to the specific form or arrangement herein described and shown. It will be apparent to those skilled in the art that various changes may be made without departing from the scope of the invention and the invention is not to be considered limited to what is shown and described in the specification. One skilled in the art will readily appreciate that the present invention is well adapted to carry out the objectives and obtain the ends and advantages mentioned, as well as those inherent therein. The excipients and related compounds described herein are presently representative of the preferred embodiments, are intended to be exemplary and are not intended as limitations on the scope. Changes therein and other uses will occur to those skilled in the art which are encompassed within the spirit of the invention and are defined by the scope of the appended claims. Although the invention has been described in connection with specific preferred embodiments, it should be understood that the invention as claimed should not be unduly limited to such specific embodiments. Indeed, various modifications of the described modes for carrying out the invention, which are obvious to those skilled in the art, are intended to be within the scope of the following claims.

## Claims

1. An oil-in-water formulation for the inhibition of the transmission of sexually transmitted diseases, **characterized by** enhanced skin protective properties consisting of in combination:
(1) a skin protectant composition selected from the group consisting of Allantoin, Aluminum hydroxide gel, Calamine, Cocoa Butter, Cod Liver Oil, Colloidal Oatmeal, Hard Fat, Kaolin, Mineral Oil, Petrolatum, Topical Starch, White Petrolatum, Zinc Acetate, Zinc Carbonate, Zinc Oxide, and mixtures thereof, provided in a skin protecting effective concentration;
(2) a silicone-based skin protectant composition containing at least one silicone-containing excipient provided in a skin protecting effective concentration;
(3) a humectant-excipient, selected from the group consisting of glycerin, anhydrous lanolin and combinations thereof, in the range of from 0.5 to 15.0 % wt/wt;
(4) a non-ionic emulsifier selected from the group consisting of cetearyl alcohol, ceteareth-12, ceteareth-20 and mixtures thereof, in the range from 0.5 to 15.0 % w/w;
(5) a chelating compound in the range from 0.1 to 0.01 % w/w;
(6) a film-forming emollient in the range from 1.0 to 10.0% w/w; and
(7) water;
wherein contact with the skin results in the *in situ* formation of a skin protective barrier layer effective in inhibiting the transmission of sexually transmitted diseases.

2. The formulation of claim 1 wherein said skin protectant is zinc oxide.

3. The formulation of claim 1, wherein said silicone-based skin protectant is Dimethicone 20, or wherein said silicone-based skin protectant is Dimethicone 12500, or wherein said silicone-based skin protectant is a combination of Dimethicone 20 and Dimethicone 12500.

4. The formulation of any one of the proceeding claims, wherein said film-forming emollient is cetostearyl alcohol.

5. The formulation of any one of the proceeding claims, wherein said film-forming emollient is cetyl alcohol, or wherein said film-forming emollient is a combination of cetostearyl alcohol and cetyl alcohol.

6. The formulation of any one of the proceeding claims, wherein said chelating compound is disodium edetate.

7. The formulation of any one of the proceeding claims, wherein said formulation is in the form of a cream, or wherein said formulation is in the form of a lotion.

8. An oil-in-water formulation according to claim 7, wherein said formulation is a cream consisting of in combination: (1) Dimethicone 20, in the range of 10.0 to 20.0% wt/wt; (2) Dimethicone 12500, in the range of 3.0-10.0% wt/wt; (3) Zinc oxide, in the range of 1.0 to 8.0% wt/wt; (4) a mixture of Cetearyl alcohol and Ceteareth-20, in the range of 6.0 to 10.0% wt/wt; (5) Glycerin, in the range of 2.0 to 10.0% wt/wt; (6) Ceteareth-12, in the range of 0.5 to 3.0% wt/wt; (7) disodium edetate, in the range of 0.01 to 0.1% wt/wt; (8) Anhydrous lanolin, in the range of 0.5 to 3.0% wt/wt; (9) Cetostearyl alcohol, in the range of 1.0 to 5.0% wt/wt; (10) Cetyl alcohol, in the range of 1.0 to 5.0% wt/wt; and (11) a sufficient quantity of deionized water to form the cream; wherein contact with the skin results in the in situ formation of a skin protective barrier layer.

9. An oil-in-water formulation according to claim 7, wherein said formulation is a cream consisting of in combination: (1) Dimethicone 20, at about 15.0% wt/wt; (2) Dimethicone 12500, at about 5.0% wt/wt; (3) Zinc oxide, at about 2.0% wt/wt; (4) a mixture of Cetearyl alcohol and Ceteareth-20, at about 8.0% wt/wt; (5) Glycerin, at about 5.0% wt/wt; (6) Ceteareth-12, at about 1.0% wt/wt; (7) disodium edetate, at about 0.05% wt/wt; (8) Anhydrous lanolin, at about 1.0% wt/wt; (9) Cetostearyl alcohol, at about 3.0% wt/wt; (10) Cetyl alcohol, at about 2.0% wt/wt; and (11) a sufficient quantity of deionized water to form the cream; wherein contact with the skin results in the in situ formation of a skin protective barrier layer.

10. An oil-in-water formulation according to claim 7, wherein said formulation is a cream consisting of in combination: (1) Dimethicone 20, at about 15.0% wt/wt; (2) Dimethicone 12500, at about 5.0% wt/wt; (3) Zinc oxide, at about 5.0% wt/wt; (4) a mixture of Cetearyl alcohol and Ceteareth-20, at about 7.0% wt/wt; (5) Glycerin, at about 8.0% wt/wt; (6) Ceteareth-12, at about 1.0% wt/wt; (7) disodium edetate, at about 0.05% wt/wt; (8) Anhydrous lanolin, at about 1.0% wt/wt; (9) Cetostearyl alcohol, at about 3.0% wt/wt; (10) Cetyl alcohol, at about 2.0% wt/wt; and (11) a sufficient quantity of deionized water to form the cream; wherein contact with the skin results in the in situ formation of a skin protective barrier layer.

11. An oil-in-water formulation according to claim 1 consisting of in combination the following ingredients in % w/w:
a water phase consisting of
| | | |
|---|---|---|
| (1) | DEIONIZED WATER (USP) in sufficient to quantities dissolve ingredients 2-4; | |
| (2) | DISODIUM EDETATE | 0.01-0.1 |
| (3) | ZINC OXIDE | 1.0-8.0 |
| (4) | GLYCERIN (USP) | 2.0-10.0 |
| and an oil phase consisting of | | |
|---|---|---|
| (5) | DIMETHICONE 20 | 10.0-20.0 |
| (6) | DIMETHICONE 12500 | 3.0-10.0 |
| (7) | CETEARYL ALCOHOL and/or CETEARETH-20 | 6.0-10.0 |
| (8) | CETEARETH-12 | 0.5-3.0 |
| (9) | ANHYDROUS LANOLIN | 0.5-3.0 |
| (10) | CETOSTEARYL ALCOHOL | 1.0-5.0 |
| (11) | CETYL ALCOHOL | 1.0-5.0 |

12. A process for forming an oil-in-water cream consisting of:
| a water phase consisting of (in % w/w) | | |
|---|---|---|
| (a) | Deionized Water (USP) in quantities sufficient to incorporate therein ingredients b-d; | |
| (b) | Disodium Edetate | 0.01-0.1 |
| (c) | Zinc Oxide | 1.0-8.0 |
| (d) | Glycerin (USP) | 2.0-10.0 |
| and an oil phase consisting of (in % w/w) | | |
|---|---|---|
| (e) | Dimethicone 20 | 10.0-20.0 |
| (f) | Dimethicone 12500 | 3.0-10.0 |
| (g) | Cetearyl Alcohol And/or Ceteareth-20 | 6.0-10.0 |
| (h) | Ceteareth-12 | 0.5-3.0 |
| (i) | Anhydrous Lanolin | 0.5-3.0 |
| (j) | Cetostearyl Alcohol | 1.0-5.0 |
| (k) Cetyl Alcohol | | 1.0-5.0 |
wherein said oil-in-water cream is produced in accordance with the following steps:
1.heating to 75°C Dimethicone 20, Dimethicone 12500, Cetearyl Alcohol, Ceteareth-20, Ceteareth-12, Anhydrous Lanolin, Cetostearyl Alcohol and Cetyl Alcohol to allow the materials to melt;
2. dissolving Disodium Edetate in the deionized water;
3. Heating Step #2 to 75-78 °C;
4. Dispersing the Zinc Oxide in the Glycerin to form a homogeneous dispersion;
5. With high speed mixing, slowly adding the materials from Step #3 (Water phase) to the materials from Step #1 (Oil phase) and initiating cooling;
6. When at 60 °C; adding the dispersion from Step#4 to the materials from Step #5;
7. When at 50 °C, reduce mixing speed;
8. When at 40 °C, further reduce the mixing speed;
9. When at 25°C stop mixing.

13. A formulation according to any one of claims 1 to 11 for use in the inhibition of transmission of a sexually transmitted disease

14. The formulation for use according to claim 13, wherein said sexually transmitted disease is a bacterial sexually transmitted disease, preferably selected from gonorrhea, chlamydia or syphilis, or said sexually transmitted disease is a viral sexually transmitted disease, preferably selected from human immunodeficiency virus (HIV), human papilloma virus (HPV) or hepatitis.

## Patentansprüche

1. Öl-in-Wasser-Formulierung zur Hemmung der Übertragung sexuell übertragener Krankheitserreger, **gekennzeichnet durch** verbesserte hautschützende Eigenschaften, bestehend aus einer Kombination aus:
(1) einer die Haut schützenden Zusammensetzung, die ausgewählt ist aus der Gruppe bestehend aus Allantoin, Aluminiumhydroxidgel, Calamin, Kakaobutter, Lebertranöl, kolloidalem Hafermehl, Hartfett, Kaolin, Mineralöl, Petrolatum, topischer Stärke, weißem Petrolatum, Zinkacetat, Zinkcarbonat, Zinkoxid und Mischungen dieser, bereitgestellt in einer die Haut schützenden Wirkkonzentration;
(2) einer die Haut schützenden Zusammensetzung auf Silikonbasis, die mindestens einen Silikon enthaltenden Träger aufweist, der in einer die Haut schützenden Wirkkonzentration vorgesehen ist;
(3) einem Feuchthaltemittelträger, der ausgewählt ist aus der Gruppe bestehend aus Glycerin, wasserfreiem Lanolin und Kombinationen dieser im Bereich von 0,5 bis 15,0 Gew.-%;
(4) einem nichtionischem Emulgator, der ausgewählt ist aus der Gruppe bestehend aus Cetylstearylalkohol, Ceteareth-12, Ceteareth-20 und Mischungen dieser im Bereich von 0,5 bis 15,0 Gew.-%;
(5) einer gelbildenden Verbindung im Bereich von 0,1 bis 0,01 Gew.-%;
(6) einem filmbildenden Weichmacher im Bereich von 1,0 bis 10,0 Gew.-%; und
(7) Wasser;
wobei ein Kontakt mit der Haut zu einer in situ-Bildung einer hautschützenden Sperrschicht führt, die wirksam die Übertragung sexuell übertragener Krankheiten hemmt.

2. Formulierung nach Anspruch 1, wobei die Haut schützende Substanz Zinkoxid ist.

3. Formulierung nach Anspruch 1, wobei die Haut schützende Substanz auf Silikonbasis Polydimethylsiloxan 20 ist, oder wobei die Haut schützende Substanz auf Silikonbasis Polydimethylsiloxan 12500 ist, oder wobei die Haut schützende Substanz auf Silikonbasis eine Kombination aus Polydimethylsiloxan 20 und Polydimethylsiloxan 12500 ist.

4. Formulierung nach einem der vorstehenden Ansprüche, wobei der filmbildende Weichmacher Cetylstearylalkohol ist

5. Formulierung nach einem der vorstehenden Ansprüche, wobei der filmbildende Weichmacher Cetylalkohol ist, oder wobei der filmbildende Weichmacher eine Kombination aus Cetylstearylalkohol und Cetylalkohol ist.

6. Formulierung nach einem der vorstehenden Ansprüche, wobei die gelbildende Verbindung Dinatriumedetat ist.

7. Formulierung nach einem der vorstehenden Ansprüche, wobei die Formulierung die Form einer Creme aufweist, oder wobei die Formulierung die Form einer Lotion aufweist.

8. ÖI-in-Wasser-Formulierung nach Anspruch 7, wobei die Formulierung eine Creme ist, bestehend aus einer Kombination aus: (1) Polydimethylsiloxan 20 im Bereich von 10,0 bis 20,0 Gew.-%; (2) Polydimethylsiloxan 12500 im Bereich von 3,0 bis 10,0 Gew.-%; (3) Zinkoxid im Bereich von 1,0 bis 8,0 Gew.-%; (4) einer Mischung aus Cetylstearylalkohol und Ceteareth-20 im Bereich von 6,0 bis 10,0 Gew.-%; (5) Glycerin im Bereich von 2,0 bis 10,0 Gew.-%; (6) Ceteareth-12 im Bereich von 0,5 bis 3,0 Gew.-%; (7) Dinatriumedetat im Bereich von 0,01 bis 0,1 Gew.-%; (8) wasserfreiem Lanolin im Bereich von 0,5 bis 3,0 Gew.-%; (9) Cetostearylalkohol im Bereich von 1,0 bis 5,0 Gew.-%; (10) Cetylalkohol im Bereich 1,0 bis 5,0 Gew.-%; und (11) einer ausreichenden Menge deionisiertes Wasser zur Bildung der Creme;;
wobei ein Kontakt mit der Haut zu einer in situ-Bildung einer hautschützenden Sperrschicht führt.

9. Öl-in-Wasser-Formulierung nach Anspruch 7, wobei die Formulierung eine Creme ist, bestehend aus einer Kombination aus: (1) Polydimethylsiloxan 20 in Höhe von etwa 15,0 Gew.-%; (2) Polydimethylsiloxan 12500 Höhe von etwa 5,0 Gew.-%; (3) Zinkoxid Höhe von etwa 2,0 Gew.-%; (4) einer Mischung aus Cetylstearylalkohol und Ceteareth-20 Höhe von etwa 8,0 Gew.-%; (5) Glycerin Höhe von etwa 5,0 Gew.-%; (6) Ceteareth-12 Höhe von etwa 1,0 Gew.-%; (7) Dinatriumedetat in Höhe von etwa 0,5 Gew.-%; (8) wasserfreiem Lanolin Höhe von etwa 1,0 Gew.-%; (9) Cetostearylalkohol Höhe von etwa 3,0 Gew.-%; (10) Cetylalkohol Höhe von etwa 2,0 Gew.-%; und (11) einer ausreichenden Menge deionisiertes Wasser zur Bildung der Creme;;
wobei ein Kontakt mit der Haut zu einer in situ-Bildung einer hautschützenden Sperrschicht führt.

10. ÖI-in-Wasser-Formulierung nach Anspruch 7, wobei die Formulierung eine Creme ist, bestehend aus einer Kombination aus: (1) Polydimethylsiloxan 20 in Höhe von etwa 15,0 Gew.-%; (2) Polydimethylsiloxan 12500 Höhe von etwa 5,0 Gew.-%; (3) Zinkoxid Höhe von etwa 5,0 Gew.-%; (4) einer Mischung aus Cetylstearylalkohol und Ceteareth-20 Höhe von etwa 7,0 Gew.-%; (5) Glycerin Höhe von etwa 8,0 Gew.-%; (6) Ceteareth-12 Höhe von etwa 1,0 Gew.-%; (7) Dinatriumedetat in Höhe von etwa 0,05 Gew.-%; (8) wasserfreiem Lanolin Höhe von etwa 1,0 Gew.-%; (9) Cetostearylalkohol Höhe von etwa 3,0 Gew.-%; (10) Cetylalkohol Höhe von etwa 2,0 Gew.-%; und (11) einer ausreichenden Menge deionisiertes Wasser zur Bildung der Creme;;
wobei ein Kontakt mit der Haut zu einer in situ-Bildung einer hautschützenden Sperrschicht führt.

11. ÖI-in-Wasser-Formulierung nach Anspruch 1, wobei diese in Kombination aus den folgenden Bestandteilen in Gew.-% besteht:
einer Wasserphase, die folgendes aufweist:
(1) deionisiertes Wasser (USP) in Mengen, die ausreichen, die Bestandteile 2-4 darin zu inkorporieren;
(2) Dinatriumedetat 0,01-0,1
(3) Zinkoxid 1,0-8,0
(4) Glycerin (USP) 2,0-10,0
und einer Ölphase, die folgendes aufweist:
(5) Polydimethylsiloxan 20 10,0-20,0
(6) Polydimethylsiloxan 12500 3,0-10,0
(7) Cetystearylalkohol und/oder Ceteareth-20 6,0-10,0
(8) Ceteareth-12 0,5-3,0
(9) Lanolin 0,5-3,0
(10) Cetostearylalkohol 1,0-5,0
(11) Cetylalkohol 1,0-5,0.

12. Verfahren zur Bildung einer ÖI-in-Wasser-Formulierung, bestehend aus:
einer Wasserphase, die folgendes aufweist (in Gew.-%):
(a) deionisiertes Wasser (USP) in Mengen, die ausreichen, die Bestandteile b-d darin zu inkorporieren;
(b) Dinatriumedetat 0,01-0,1
(c) Zinkoxid 1,0-8,0
(d) Glycerin (USP) 2,0-10,0
und einer Ölphase, die folgendes aufweist (in Gew.-%):
(e) Polydimethylsiloxan 20 10,0-20,0
(f) Polydimethylsiloxan 12500 3,0-10,0
(g) Cetystearylalkohol und/oder Ceteareth-20 6,0-10,0
(h) Ceteareth-12 0,5-3,0
(i) Lanolin 0,5-3,0
(j) Cetostearylalkohol 1,0-5,0
(k) Cetylalkohol 1,0-5,0;
wobei die Öl-in-Wasser-Formulierung gemäß den folgenden Schritten erzeugt wird:
1) Erhitzen von Polydimethylsiloxan 20, Polydimethylsiloxan 12500, Cetystearylalkohol, Ceteareth-20, Ceteareth-12, wasserfreiem Lanolin, Cetostearylalkohol und Cetylalkohol auf 75 °C, um ein Schmelzen der Substanzen zu ermöglichen;
2) Auflösen von Dinatriumedetat in deonisiertem Wasser;
3) Erhitzen von Schritt #2 auf 75-78 °C;
4) Dispergieren des Zinkoxids in dem Glycerin, so dass eine homogene Dispersion gebildet wird;
5) Unter Hochgeschwindigkeitsmischen langsames Hinzufügen der Substanzen aus Schritt #3 (Wasserphase) zu den Substanzen aus Schritt #1 (Ölphase) und Einleiten des Kühlens;
6) beim Erreichen von 60 °C, Hinzufügen der Dispersion aus Schritt #4 zu den Substanzen aus Schritt #5;
7) beim Erreichen von 50 °C, Verringern der Mischgeschwindigkeit;
8) beim Erreichen von 40 °C, weiteres Verringern der Mischgeschwindigkeit;
9) beim Erreichen von 25 °C, Einstellen des Mischens.

13. Formulierung nach einem der Ansprüche 1 bis 11 zum Einsatz im Hemmen der Übertragung einer sexuell übertragenen Krankheit.

14. Formulierung zum Einsatz nach Anspruch 13, wobei die sexuell übertragene Krankheit eine bakterielle sexuell übertragene Krankheit ist, die vorzugsweise ausgewählt ist aus Gonorrhö, Chlamydien oder Syphillis, oder wobei die sexuell übertragene Krankheit eine virale sexuell übertragene Krankheit ist, die vorzugsweise ausgewählt ist aus Humanes Immundefizienz-Virus (HIV), Humanes Papilloma Virus (HPV) oder Hepatitis.

## Revendications

1. Formulation huile dans eau pour l'inhibition de la transmission de maladies sexuellement transmissibles, **caractérisée par** des propriétés de protection de la peau améliorées, consistant en une combinaison de :
(1) une composition de protection de la peau choisie dans le groupe constitué par l'allantoïne, le gel d'hydroxyde d'aluminium, la calamine, le beurre de cacao, l'huile de foie de morue, la farine d'avoine colloïdale, la graisse dure, le kaolin, une huile minérale, le pétrolatum, l'amidon topique, le pétrolatum blanc, l'acétate de zinc, le carbonate de zinc, l'oxyde de zinc et leurs mélanges, en une concentration efficace pour la protection de la peau ;
(2) une composition de protection de la peau à base de silicone contenant au moins un excipient contenant de la silicone en une concentration efficace pour la protection de la peau ;
(3) un excipient humectant, choisi dans le groupe constitué par la glycérine, la lanoline anhydre et leurs combinaisons, dans la plage allant de 0,5 à 15,0 % en poids ;
(4) un émulsifiant non ionique choisi dans le groupe constitué par l'alcool cétéarylique, le cétéareth 12, le cétéareth 20 et leurs mélanges, dans la plage allant de 0,5 à 15,0 % en poids ;
(5) un composé chélateur, dans la plage allant de 0,1 à 0,01 % en poids ;
(6) un émollient filmogène, dans la plage allant de 1,0 à 10,0 % en poids ; et
(7) de l'eau ;
un contact avec la peau résultant en la formation *in situ* d'une couche de barrière de protection de l'eau efficace pour inhiber la transmission de maladies sexuellement transmissibles.

2. Formulation selon la revendication 1, dans laquelle ledit agent de protection de la peau est l'oxyde de zinc.

3. Formulation selon la revendication 1, dans laquelle ledit agent de protection de la peau à base de silicone est la diméthicone 20, ou dans laquelle ledit agent de protection de la peau à base de silicone est la diméthicone 12500, ou dans laquelle ledit agent de protection de la peau à base de silicone est une combinaison de diméthicone 20 et de diméthicone 12500.

4. Formulation selon l'une quelconque des revendications précédentes, dans laquelle ledit émollient filmogène est l'alcool cétostéarylique.

5. Formulation selon l'une quelconque des revendications précédentes, dans laquelle ledit émollient filmogène est l'alcool cétylique, ou dans laquelle ledit émollient filmogène est une combinaison d'alcool cétostéarylique et d'alcool cétylique.

6. Formulation selon l'une quelconque des revendications précédentes, dans laquelle ledit composé chélateur est l'édétate de disodium.

7. Formulation selon l'une quelconque des revendications précédentes, dans laquelle ladite formulation est sous la forme d'une crème ou dans laquelle ladite formulation est sous la forme d'une lotion.

8. Formulation huile dans eau selon la revendication 7, dans laquelle ladite formulation est une crème constituée par une combinaison de : (1) diméthicone 20, dans la plage allant de 10,0 à 20,0 % en poids, (2) diméthicone 12500, dans la plage allant de 3,0 à 10,0 % en poids ; (3) oxyde de zinc, dans la plage allant de 1,0 à 8,0 % en poids ; (4) un mélange d'alcool cétéarylique et de cétéareth 20, dans la plage allant de 6,0 à 10,0 % en poids ; (5) glycérine, dans la plage allant de 2,0 à 10,0 % en poids ; (6) cétéareth 12, dans la plage allant de 0,5 à 3,0 % en poids ; (7) édétate de disodium, dans la plage allant de 0,01 à 0,1 % en poids ; (8) lanoline anhydre, dans la plage allant de 0,5 à 3,0 % en poids ; (9) alcool cétostéarylique, dans la plage allant de 1,0 à 5,0 % en poids ; (10) alcool cétylique, dans la plage allant de 1,0 à 5,0 % en poids ; et (11) une quantité suffisante d'eau déionisée pour former la crème ; un contact avec la peau résultant en la formation *in situ* d'une couche de barrière de protection de la peau.

9. Formulation huile dans eau selon la revendication 7, dans laquelle ladite formulation est une crème constituée par une combinaison de : (1) diméthicone 20, à hauteur d'environ 15,0 % en poids, (2) diméthicone 12500, à hauteur d'environ 5,0 % en poids ; (3) oxyde de zinc, à hauteur d'environ 2,0 % en poids ; (4) un mélange d'alcool cétéarylique et de cétéareth 20, à hauteur d'environ 8,0 % en poids ; (5) glycérine, à hauteur d'environ 5,0 % en poids ; (6) cétéareth 12, à hauteur d'environ 1,0 % en poids ; (7) édétate de disodium, à hauteur d'environ 0,05 % en poids ; (8) lanoline anhydre, à hauteur d'environ 1,0 % en poids ; (9) alcool cétostéarylique, à hauteur d'environ 3,0 % en poids ; (10) alcool cétylique, à hauteur d'environ 2,0 % en poids ; et (11) une quantité suffisante d'eau déionisée pour former la crème ; un contact avec la peau résultant en la formation *in situ* d'une couche de barrière de protection de la peau.

10. Formulation huile dans eau selon la revendication 7, dans laquelle ladite formulation est une crème constituée par une combinaison de : (1) diméthicone 20, à hauteur d'environ 15,0 % en poids, (2) diméthicone 12500, à hauteur d'environ 5,0 % en poids ; (3) oxyde de zinc, à hauteur d'environ 5,0 % en poids ; (4) un mélange d'alcool cétéarylique et de cétéareth 20, à hauteur d'environ 7,0 % en poids ; (5) glycérine, à hauteur d'environ 8,0 % en poids ; (6) cétéareth 12, à hauteur d'environ 1,0 % en poids ; (7) édétate de disodium, à hauteur d'environ 0,05 % en poids ; (8) lanoline anhydre, à hauteur d'environ 1,0 % en poids ; (9) alcool cétostéarylique, à hauteur d'environ 3,0 % en poids ; (10) alcool cétylique, à hauteur d'environ 2,0 % en poids ; et (11) une quantité suffisante d'eau déionisée pour former la crème ; un contact avec la peau résultant en la formation *in situ* d'une couche de barrière de protection de la peau.

11. Formulation huile dans eau selon la revendication 1, consistant en une combinaison des ingrédients suivants en % en poids :
une phase aqueuse constituée par :
(1) EAU DÉIONISÉE (USP) en quantités suffisantes pour dissoudre les ingrédients 2 à4;
(2) ÉDÉTATE DE DISODIUM 0,01 à 0,1
(3) OXYDE DE ZINC 1,0 à 8,0
(4) GLYCÉRINE (USP) 2,0 à 10,0
et une phase huileuse constitué par :
(5) DIMÉTHICONE 20 10,0 à 20,0
(6) DIMÉTHICONE 12500 3,0 à 10,0
(7) ALCOOL CÉTÉARYLIQUE et/ou CÉTÉARETH 20 6,0 à 10,0
(8) CÉTÉARETH 12 0,5 à 3,0
(9) LANOLINE ANHYDRE 0,5 à 3,0
(10) ALCOOL CÉTOSTÉARYLIQUE 1,0 à 5,0
(11) ALCOOL CÉTYLIQUE 1,0 à 5,0.

12. Procédé de formation d'une crème huile dans eau constituée par :
une phase aqueuse constituée par (en % en poids) :
(a) eau déionisée (USP) en quantités suffisantes pour y incorporer les ingrédients b à d ;
(b) édétate de disodium 0,01 à 0,1
(c) oxyde de zinc 1,0 à 8,0
(d) glycérine (USP) 2,0 à 10,0
et une phase huileuse constitué par (en % en poids) :
(e) diméthicone 20 10,0 à 20,0
(f) diméthicone 12500 3,0 à 10,0
(g) alcool cétéarylique et/ou cétéareth 20 6,0 à 10,0
(h) cétéareth 12 0,5 à 3,0
(i) lanoline anhydre 0,5 à 3,0
(j) alcool cétostéarylique 1,0 à 5,0
(k) alcool cétylique 1,0 à 5,0,
ladite crème huile dans eau étant fabriquée selon les étapes suivantes :
1. le chauffage à 75 °C de la diméthicone 20, de la diméthicone 12500, de l'alcool cétéarylique, du cétéareth 20, du cétéareth 12, de la lanoline anhydre, de l'alcool cétostéarylique et de l'alcool cétylique pour que les matériaux fondent ;
2. la dissolution de l'édétate de disodium dans l'eau déionisée ;
3. le chauffage de l'étape #2 à une température de 75 à 78 °C ;
4. la dispersion de l'oxyde de zinc dans la glycérine pour former une dispersion homogène ;
5. sous mélange rapide, l'ajout lent des matériaux de l'étape #3 (phase aqueuse) aux matériaux de l'étape #1 (phase huileuse) et l'initiation du refroidissement ;
6. à 60 °C, l'ajout de la dispersion de l'étape #4 aux matériaux de l'étape #5 ;
7. à 50 °C, la réduction de la vitesse de mélange ;
8. à 40 °C, la réduction supplémentaire de la vitesse de mélange ;
9. à 25 °C, l'arrêt du mélange.

13. Formulation selon l'une quelconque des revendications 1 à 11, destinée à une utilisation pour inhiber la transmission d'une maladie sexuellement transmissible.

14. Formulation pour une utilisation selon la revendication 13, dans laquelle ladite maladie sexuellement transmissible est une maladie bactérienne sexuellement transmissible, de préférence choisie dans le groupe constitué par la gonorrhée, la chlamydia ou la syphilis, ou ladite maladie sexuellement transmissible est une maladie virale sexuellement transmissible, de préférence choisie parmi le virus de l'immunodéficience humaine (VIH), le virus du papillome humain (VPH) ou l'hépatite.
